# EUROPÄISCHE PATENTSCHRIFT

(11) **EP 0 643 030 B1**
(45) Veröffentlichungstag und Bekanntmachung des Hinweises auf die Patenterteilung: **04.06.1997**
(21) Anmeldenummer: 94113438.9
(22) Anmeldetag: 29.08.1994
(51) Int. Cl.: C07C 17/38, C07C 25/08

(54) **Verfahren zur Isolierung von m-Dichlorbenzol aus Dichlorbenzol-Isomerengemischen**
Process for isolating m-dichlorobenzene from isomeric dichlorobenzene mixtures
Procédé d'isolation de m-dichlorobenzène de mélanges isomères de dichlorobenzènes

(30) Priorität: 10.09.1993 DE 4330731
(43) Veröffentlichungstag der Anmeldung: 15.03.1995
(73) Patentinhaber: BAYER AG, 51368 Leverkusen (DE)
(72) Erfinder: Pies, Dr. Michael, D-47249 Duisburg (DE); Röhlk, Dr. Kai, D-51467 Bergisch Gladbach (DE); Lahr, Dr. Helmut, D-51519 Odenthal (DE); Dr. Fiege, Helmut, D-51373 Leverkusen (DE)

(56) Entgegenhaltungen:
- DE-A- 3 617 137
- FR-A- 2 069 748

## Beschreibung

Die vorliegende Erfindung betrifft ein besonders vorteilhaftes Verfahren zur Isolierung von m-Dichlorbenzol (m-DCB) aus Dichlorbenzol-Isomerengemischen.

m-DCB ist ein wichtiges Zwischenprodukt, beispielsweise zur Herstellung von Pharmazeutika und Pflanzenschutzmitteln.

Dichlorbenzole werden üblicherweise durch Chlorierung von Benzol bzw. Mono-chlorbenzol hergestellt. In Abhängigkeit von den angewendeten Reaktionsbedingungen und Katalysatoren erhält man unterschiedlich zusammengesetzte Isomerengemische, die beispielsweise 55 - 80 Gew.-% p-DCB, 1 - 3 Gew.-% m-DCB und 20 - 25 Gew.-% o-DCB enthalten können (siehe Ullmanns Encyclopedia of Industrial Chemistry, 5. Auflage, Band A 6, Seite 333 ff.).

Eine Steigerung des m-DCB-Gehaltes im Isomerengemisch ist z.B. durch Isomerisierung von o- und p-DCB an Katalysatoren möglich, beispielsweise an Aluminiumtrichlorid oder Zeolithen. So kann man Dichlorbenzol-Isomerengemische mit einem m-DCB-Gehalt von bis zu 60 Gew.-% erhalten.

Für die Auftrennung von Dichlorbenzol-Isomerengemischen, insbesondere m-/p-DCB-Gemischen, in die jeweils reinen DCB-Isomere sind verschiedene Verfahren bekannt.

Aufgrund der sehr geringen Siedepunktdifferenzen der einzelnen DCB-Isomeren untereinander (o-DCB: 180°C; m-DCB: 173°C; p-DCB: 174,1°C) kann durch Destillation mit vertretbarem Aufwand nur eine Trennung in o-DCB (Sumpfprodukt) und ein m-/p-DCB-Gemisch (Kopfprodukt) bewerkstelligt werden.

Es sind auch schon extrahierende Destillationsverfahren vorgeschlagen worden (siehe z.B. EP-A 451 720). Dafür werden oft schwierig zugängliche und daher teure Extraktionsmittel benötigt (z.B. Alkylencarbonate), ohne daß eine befriedigende Trennung zwischen m-DCB und p-DCB erzielt werden können.

Durch Sulfierung oder Bromierung von m-/p-DCB-Gemischen kann man recht selektiv m-DCB in Sulfo- bzw. Brom-m-DCB überführen, die dann leicht vom unumgesetzten p-DCB abgetrennt werden können. Nachteilig ist hier die anschließend durchzuführende Desulfierung oder Debromierung, um wieder m-DCB zu erhalten.

Eine Kombination von Kristallisationen und Destillationen (siehe DE-B1 2 855 940) erlaubt zwar eine m-DCB-Anreicherung bis hin zur eutektischen Mischung (ca. 85 Gew.-% m-DCB / ca. 15 Gew.-% p-DCB) die Destillation erfordert aber einen hohen Energie- und Zeitaufwand und ist somit für eine industrielle Nutzung nicht besonders vorteilhaft.

Bekannt ist ferner, daß p-DCB mit 1-Brom-4-Chlorbenzol sowie 1,4-Dibrombenzol eutektische Kristalle bildet. Deshalb wurde in der DE-A 3 617 137 vorgeschlagen, diese Substanzen zu m-/p-DCB-Gemischen zuzusetzen und durch Kristallisation p-DCB in Form von eutektischen Kristallen zu entfernen, die p-DCB und 1-Brom-4-Chlorbenzol oder 1,4-Dibrombenzol enthalten. Technisch interessant sind nur solche Kristallisationen, die ausgehend von eutektischen Mischungen m-DCB in Reinheiten von über 90 Gew.-% liefern. Mit 1-Brom-4-Chlorbenzol und 1,4-Dibrombenzol gelingt dies nur bei Temperaturen im Bereich von -10 bis -24°C (siehe DE-OS 2 855 940, Beispiel 20 und 21), was einen erheblichen technischen und energetischen Aufwand bedingt.

Die sich an die Kristallisation anschließende Trennung der Komponenten der eutektischen Kristalle kann durch Destillation erfolgen. Je größer der Abstand der Siedepunkte der Komponenten der eutektischen Kristalle ist, um so schneller und leichter können sie voneinander getrennt werden. Das gemäß der DE-OS 3 617 137 einzusetzende 1-Brom-4-Chlorbenzol siedet bei 196°C. Es ist deshalb nur mit erheblichem Aufwand von p-DCB zu trennen. Außerdem ist es zur Gewinnung von reinem m-DCB sowieso prinzipiell von Vorteil, das m-DCB zu kristallisieren (und nicht das p-DCB), da Verunreinigungen im eingesetzten m-/p-DCB-Gemisch bei der Kristallisation normalerweise in der Mutterlauge verbleiben und nicht oder nur in geringem Umfang in das Kristallisat gelangen.

Es besteht daher immer noch ein Bedürfnis nach einem Verfahren, durch das m-DCB in möglichst reiner Form auf einfache Weise zugänglich wird.

Es wurde nun ein Verfahren zur Isolierung von m-DCB aus Dichlorbenzol-Isomerengemischen durch Kristallisation als eutektische Kristalle mit einem Hilfsstoff gefunden, das dadurch gekennzeichnet ist, daß man als Hilfsstoff eine Verbindung der Formel (I) einsetzt 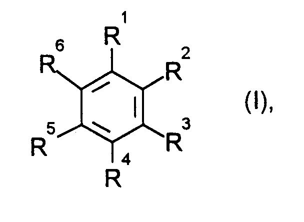 in der
- R¹ bis R⁶: unabhängig voneinander für Wasserstoff, Halogen, C₁-C₄-Alkyl, Hydroxyl, NH₂ oder R-CO- mit R = C₁-C₄-Alkyl stehen,
wobei 1-Brom-4-Chlorbenzol und 1,4-Dibrombenzol ausgenommen sind.

Halogen kann beispielsweise für Fluor, Chlor oder Brom stehen, vorzugsweise steht es für Chlor.

Vorzugsweise stehen in Formel (I) mindestens 2 der Reste R¹ bis R⁶ für Wasserstoff und bis zu vier dieser Reste für Chlor oder C₁- bis C₄-Alkyl, wobei 1,4-Di-brombenzol ausgenommen ist.

Besonders bevorzugt als Hilfsstoffe sind Tetrachlorbenzole und Kresole, insbesondere 1,2,3,4-Tetrachlorbenzol und p-Kresol.

Zum Einsatz in das erfindungsgemäße Verfahren eignen sich beliebige Dichlorbenzol-Isomerengemische. Vorzugsweise werden Gemische eingesetzt, wie man sie nach der Dichiorierung von Benzol, anschließender katalytischer Isomerisierung und destillativer Abtrennung der Hauptmenge o-DCB erhalten kann. Bevorzugt einzusetzende Dichlorbenzol-Isomerengemische können z.B. 80 - 86 Gew.-% m-DCB, 14 - 20 Gew.-% p-DCB und gegebenenfalls bis zu 2 Gew.-% andere Stoffe enthalten.

Besonders bevorzugt sind Isomerengemische, aus denen o-DCB bereits durch Destillation abgetrennt wurde.

Hilfsstoffe der Formel (I) können beispielsweise in Mengen von 20 - 200 Gew.-Teilen pro 100 Gew.-Teile Dichlorbenzol-Isomerengemisch eingesetzt werden. Vorzugsweise beträgt die Menge 50 bis 130 Gew.-Teile pro 100 Gew.-Teile Dichlorbenzol-Isomerengemisch.

Die Qualitätsanforderung an den eingesetzten Hilfsstoff ist nicht hoch. Beispielsweise kann 1,2,3,4-Tetrachlorbenzol eingesetzt werden, wie es durch Chlorierung von 1,2,3-Trichlorbenzol gewonnen wird, d.h. es können auch geringe Mengen 1,2,3-Trichlorbenzol und/oder andere Tetrachlorbenzolisomeren enthalten sein. Besonders geeignet ist 1,2,3,4-Tetrachlorbenzol mit einer Reinheit von über 90%.

Im eingesetzten Hilfsstoff enthaltene Verunreinigungen finden sich nach Durchführung des erfindungsgemäßen Verfahrens im allgemeinen in der Mutterlauge, sodaß nach Trennung der Komponenten der ausgefallenen eutektischen Kristalle aus m-DCB und Hilfsstoff nicht nur das m-DCB, sondern auch der Hilfsstoff in reiner Form vorliegt.

Das erfindungsgemäße Verfahren kann man beispielsweise so durchführen, daß man zunächst den Hilfsstoff in einem Dichlorbenzol-Isomerengemisch löst, gegebenenfalls unter leichtem Erwärmen, und in einer an sich bekannten, kontinuierlich oder diskontinuierlich arbeitenden Kristallisationsapparatur abkühlt. Die Kristallisationstemperatur liegt dabei im allgemeinen unter 20°C, vorzugsweise im Bereich -10 bis +10°C.

Von der nach der Kristallisation vorliegenden Aufschlämmung können die Flüssiganteile (= Mutterlauge) auf übliche Weise entfernt werden, beispielsweise durch Abblasen mit einem Gas, Filtration, Dekantieren und/oder Zentrifugieren. Nach Aufwärmung der zurückgebliebenen Kristalle auf Raumtemperatur können diese aus der Kristallisationsapparatur entnommen werden.

Aus beiden abgetrennten Fraktionen (Mutterlauge und wieder geschmolzene Kristalle) können durch einfache Destillation Dichlorbenzole vom Hilfsstoff abgetrennt und der Hilfsstoff erneut eingesetzt werden. Aus den wieder aufgeschmolzenen Kristallen erhält man m-DCB in einer Reinheit von im allgemeinen über 94 Gew.-% und aus der Destillation der Mutterlauge m-/p-DCB-Gemische, die erneut dem erfindungsgemäßen Verfahren zugeführt werden können.

Das erfindungsgemäße Verfahren hat eine Reihe von Vorteilen: So sind die einzusetzenden Hilfsstoffe leicht und kostengünstig verfügbar, sie weisen Siedepunkte auf die eine Abtrennung aus Gemischen mit Dichlorbenzolen durch eine einfache Destillation ermöglicht und sie gestalten den Kristallisationsvorgang (Auskristallisieren der eutektischen Gemische aus m-DCB und Hilfsstoff) bei höheren Temperaturen als bisher. Sie sind damit technisch einfacher und wirtschaftlicher durchzuführen als bekannte Verfahren.

### Beispiele

### Beispiel 1 (siehe auch Tabelle 1)

In einem doppelwandigen Glaszylinder (500 mm x 17 mm) wurden 138 g des Gemisches aus verschiedenen Dichlorbenzolen und verunreinigtem 1,2,3,4-Tetrachlorbenzol auf +5°C abgekühlt. Nach Einsetzen der Kristallisation stieg die Temperatur auf bis zu +8°C an. Innerhalb 1 Stunde breiteten sich die ausgefallenen Kristalle über den gesammten Kristallerraum aus. Anschließend wurde der Glaszylinder nach unten geöffnet und die Mutterlauge ablaufen gelassen. Nach Ablauf der Mutterlauge wurde die Kühlung abgestellt und bei Raumtemperatur liefen dann die ausgefallenen Kristalle in geschmolzener Form ab. Die so erhaltene Kristallschmelze enthielt m-DCB in 96,5 gew.-%iger Reinheit, bezogen auf vorliegende Dichlorbenzole. Weitere Einzelheiten sind in der Tabelle 1 angegeben. Die dort enthaltenen Angaben in Gew.-% wurden gaschromatographisch ermittelt.

### Beispiel 2 (siehe auch Tabelle 2)

In einem 50 ml fassenden Glaszylinder wurden 10 g des Gemisches aus verschiedenen Dichlorbenzolen und p-Kresol auf - 10°C abgekühlt. Die durch Rühren mit einem Glasstab spontan entstandenen Kristalle wurden durch Abnutschen von der Mutterlauge befreit. Nach Abstellen der Kühlung wurden 5 g einer Kristallschmelze erhalten. Die weiteren Einzelheiten sind der Tabelle 2 zu entnehmen. Die dort enthaltenen Angaben in Gew.-% wurden gaschromatographisch ermittelt.

**Tabelle 1**

| | eingesetztes Gemisch 138 g | abgetrennte Mutterlauge 58,7 g | Kristall schmelze 79,3 g |
|---|---|---|---|
| m-DCB(Gew.-%) | 40,2 | 42,4 | 38,2 |
| p-DCB(Gew.-%) | 6,8 | 14,0 | 1,4 |
| Gew.-Verhältnis m-/p-DCB | 85:15 | 75:25 | 96,5:3,5 |
| 1,2,3-Trichlorbenzol(Gew.-%) | 0,4 | 0,7 | 0,15 |
| 1,2,3,4-Tetrachlorbenzol(Gew.-%) | 52,3 | 42,0 | 60,2 |
| andere Tetrachlorbenzole(Gew.-%) | 0,37 | 0,86 | <0,2 |

**Tabelle 2**

| | eingesetztes Gemisch 10 g | abgetrennte Mutterlauge 5 g | Kristall schmelze 5 g |
|---|---|---|---|
| m-DCB (Gew.-%) | 42,5 | 45,2 | 39,6 |
| p-DCB (Gew.-%) | 7,5 | 12,4 | 2,6 |
| Gew.-Verhältnis m-/p-DCB | 85:15 | 78,5:21,5 | 94:6 |
| p-Kresol | 50 | 41,9 | 57,8 |

## Patentansprüche

1. Verfahren zur Isolierung von m-Dichlorbenzol aus Dichlorbenzol-Isomerengemischen durch Kristallisation als eutektische Kristalle mit einem Hiltsstoff, dadurch gekennzeichnet, daß man als Hilfsstoff eine Verbindung der Formel (I) einsetzt, 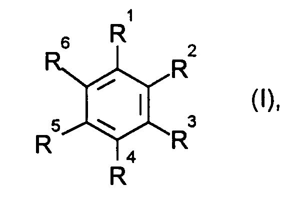 in der
R¹ bis R⁶ unabhängig voneinander für Wasserstoff, Halogen, C₁-C₄-Alkyl, Hydroxyl, NH₂ oder R-CO- mit R = C₁-C₄-Alkyl stehen,
wobei 1-Brom-4-Chlorbenzol und 1,4-Dibrombenzol ausgenommen sind.

2. Verfahren nach Anspruch 1, dadurch gekennzeichnet, daß in Formel (I) mindestens 2 der Reste R¹ bis R⁶ für Wasserstoff und bis zu 4 dieser Reste für Chlor oder C₁-C₄-Alkyl stehen, wobei 1,4-Dibrombenzol ausgenommen ist.

3. Verfahren nach Ansprüchen 1 und 2, dadurch gekennzeichnet, daß Tetrachlorbenzole oder Kresole als Hilfsstoffe eingesetzt werden.

4. Verfahren nach Ansprüchen 1 - 3, dadurch gekennnzeichnet, daß man 20 bis 200 Gew.-Teile eines Hilfsstoffes pro 100 Gew.-Teile Dichlorbenzol-Isomerengemisch einsetzt.

5. Verfahren nach Ansprüchen 1 - 4, dadurch gekennzeichnet, daß man als Dichlorbenzol-Isomerengemische solche einsetzt, die 80 - 86 Gew.-% m-DCB, 14 - 20 Gew.-% p-DCB und gegebenenfalls bis zu 2 Gew.-% andere Stoffe enthalten.

6. Verfahren nach Ansprüchen 1 - 5, dadurch gekennzeichnet, daß die Kristallisationstemperatur unter +20°C liegt.

7. Verfahren nach Ansprüchen 1 - 6, dadurch gekennzeichnet, daß die Kristallisationstemperatur im Bereich -10°C bis +15°C liegt.

8. Verfahren nach Ansprüchen 1 - 7, dadurch gekennzeichnet, daß man nach der Kristallisation die Mutterlauge auffängt und separat davon die geschmolzenen eutektischen Kristalle.

9. Verfahren nach Ansprüchen 1 - 8, dadurch gekennzeichnet, daß man aus der Mutterlauge und dem geschmolzenen Gemisch der eutektischen Kristalle den verwendeten Hilfsstoff durch Destillation abtrennt und in das erfindungsgemäße Verfahren zurückführt.

10. Verfahren nach Ansprüchen 8 und 9, dadurch gekennzeichnet, daß man aus der Mutterlauge und den geschmolzenen eutektischen Kristallen die darin enthaltenen Anteile an Dichlorbenzol abtrennt, wobei man aus der Mutterlauge ein m-/p-Dichlorbenzolgemisch erhält, das erneut dem erfindungsgemäßen Verfahren zugeführt wird und aus den geschmolzenen eutektischen Kristallen m-Dichlorbenzol mit einer Reinheit von über 90 Gew.-% erhält.

## Claims

1. Process for isolating m-dichlorobenzene from mixtures of dichlorobenzene isomers by crystallization as eutectic crystals using a processing aid, characterized in that the processing aid used is a compound of the formula (I) 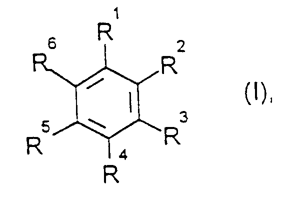 in which
R¹ to R⁶ are, independently of one another, hydrogen, halogen, C₁-C₄-alkyl, hydroxyl, NH₂ or R-CO- having R = C₁-C₄-alkyl,
with 1-bromo-4-chlorobenzene and 1,4-dibromobenzene being excepted.

2. Process according to Claim 1, characterized in that in formula (I) at least 2 of the radicals R¹ to R⁶ are hydrogen and up to 4 of these radicals are chlorine or C₁-C₄-alkyl, with 1,4-dibromobenzene being excepted.

3. Process according to Claims 1 and 2, characterized in that tetrachlorobenzenes or cresols are used as processing aids.

4. Process according to Claims 1 - 3, characterized in that from 20 to 200 parts by weight of a processing aid are used per 100 parts by weight of mixture of dichlorobenzene isomers.

5. Process according to Claims 1 - 4, characterized in that the mixtures of dichlorobenzene isomers used are ones containing 80 - 86 % by weight of m-DCB, 14 - 20 % by weight of p-DCB and optionally up to 2 % by weight of other materials.

6. Process according to Claims 1 - 5, characterized in that the crystallization temperature is below +20°C.

7. Process according to Claims 1 - 6, characterized in that the crystallization temperature is in the range from -10°C to +15°C.

8. Process according to Claims 1 - 7, characterized in that, after crystallization, the mother liquor is collected and the molten eutectic crystals are collected separately therefrom.

9. Process according to Claims 1 - 8, characterized in that the processing aid used is separated off from the mother liquor and the molten mixture of the eutectic crystals by distillation and is recirculated to the process of the invention.

10. Process according to Claims 8 and 9, characterized in that the proportions of dichlorobenzene present in the mother liquor and the molten eutectic crystals are separated therefrom, with the mother liquor giving an m-/p-dichlorobenzene mixture which is again fed to the process of the invention and the molten eutectic crystals giving m-dichlorobenzene having a purity of above 90 % by weight.

## Revendications

1. Procédé pour l'isolation du m-dichlorobenzène de mélanges d'isomères du dichlorobenzène par cristallisation sous forme de cristaux eutectiques à l'aide d'une substance auxiliaire, caractérisé en ce qu'on met en oeuvre, comme substance auxiliaire, un composé répondant à la formule (I) 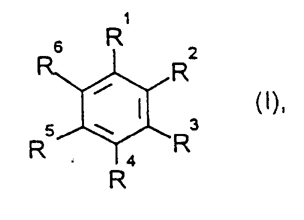 dans laquelle
R¹ à R⁶ représentent, indépendamment l'un de l'autre, un atome d'hydrogène, un atome d'halogène, un groupe alkyle en C₁-C₄, un groupe hydroxyle, NH₂ ou R-CO- où R représente un groupe alkyle en C₁-C₄,
le 1-bromo-4-chlorobenzène et le 1,4-dibromobenzène étant exclus.

2. Procédé selon la revendication 1, caractérisé en ce que, dans la formule (I), au moins deux des radicaux R¹ à R⁶ représentent un atome d'hydrogène et jusqu'à quatre de ces radicaux représentent un atome de chlore ou un groupe alkyle en C₁-C₄, le 1,4-dibromobenzène étant exclu.

3. Procédé selon les revendications 1 et 2, caractérisé en ce qu'on met en oeuvre des tétrachlorobenzènes ou des crésols comme substances auxiliaires.

4. Procédé selon les revendications 1-3, caractérisé en ce qu'on met en oeuvre de 20 à 200 parties en poids d'une substance auxiliaire par 100 parties en poids du mélange d'isomères du dichlorobenzène.

5. Procédé selon les revendications 1-4, caractérisé en ce qu'on met en oeuvre, comme mélanges d'isomères du dichlorobenzène, ceux qui contiennent du m-DCB à concurrence de 80-86% en poids, du p-DCB à concurrence de 14-20% en poids et, le cas échéant, d'autres substances jusqu'à concurrence de 2% en poids.

6. Procédé selon les revendications 1-5, caractérisé en ce que la température de cristallisation est inférieure à +20°C.

7. Procédé selon les revendications 1-6, caractérisé en ce que la température de cristallisation se situe dans le domaine de -10°C à +15°C.

8. Procédé selon les revendications 1-7, caractérisé en ce que, après la cristallisation, on récupère la liqueurmère et, séparément à cette dernière, les cristaux eutectiques portés à fusion.

9. Procédé selon les revendications 1-8, caractérisé en ce qu'on sépare par distillation de la liqueur-mère et du mélange porté à fusion des cristaux eutectiques la substance auxiliaire utilisée et on la recycle dans le procédé selon l'invention.

10. Procédé selon les revendications 8 et 9, caractérisé en ce qu'on sépare de la liqueur-mère et des cristaux eutectiques portés à fusion les fractions de dichlorobenzène qui y sont contenues de telle sorte que l'on obtient, à partir de la liqueur-mère, un mélange de m-/p-dichlorobenzène que l'on recycle à nouveau dans le procédé selon l'invention, et que l'on obtient à partir des cristaux eutectiques portés à fusion du m-dichlorobenzène possédant une pureté supérieure à 90% en poids.
